# EUROPEAN PATENT APPLICATION

(11) **EP 3 650 532 A1**
(43) Date of publication of application: **13.05.2020**
(21) Application number: 18204620.1
(22) Date of filing: 06.11.2018
(51) Int. Cl.: C12N 1/06, C12Q 1/6806, C12Q 1/686

(54) **METHODS, APPARATUS AND KITS FOR BACTERIAL CELL LYSIS**

(71) Applicant: Technische Universität Wien, 1040 Wien (AT)
(72) Inventor: REISCHER, Georg, 1140 Vienna (AT); MARTZY, Roland, 1140 Vienna (AT); SCHRÖDER, Katharina, 1020 Vienna (AT)
(74) Representative: Sonn & Partner Patentanwälte

(57) **Abstract**

The invention relates to a method for lysing a bacterial cell comprising the steps of: providing a sample comprising the bacterial cell, and adding a lysis agent to create a lysis reaction mixture; wherein the lysis agent comprises a water-miscible ionic liquid containing choline. The invention further provides an apparatus for carrying out the method as well as a kit for nucleic acid amplification from a bacterial cell and a kit for nucleic acid isolation from a bacterial cell.

## Description

The field of the present invention is the field of bacterial cell lysis.

The lysis of bacterial cells is a process involved in a wide range of applications, e.g. for the isolation and analysis of intracellular components such as nucleic acids (NAs) or proteins. The specific detection of nucleic acids from microorganisms is for example used to detect human pathogens in clinical and environmental samples, faecal indicator bacteria in water, or harmful microbial agents in food and feed. However, to detect the desired sequence of a certain NA (DNA or RNA), preceding steps are necessary to isolate the genetic material from the respective cells. These steps typically involve the lysis of the cells, the purification of the NAs to remove inhibitory substances or degrading enzymes, and the subsequent recovery of the desired NAs. Common methods for cell lysis involve thermal, chemical, enzymatic, or mechanical treatment of the cells or a combination of those (Barbosa, Cristina, et al. "DNA extraction: finding the most suitable method." Molecular Microbial Diagnostic Methods. 2016. 135-154). The purification of the NAs is, in most cases, achieved either by precipitation followed by several washing steps, or in the course of column-based purification protocols. Traditional extraction procedures for microorganisms either employ hazardous chemicals such as phenol and chloroform, or commercial kits are used, depending on the area of application and the matrix in which the cells are investigated. These methods are well established and result in high quality DNA or RNA, but they are often very laborious, time-consuming and cost-intensive, or suffer from insufficient and inconsistent yields of NAs. These disadvantages are even more significant when considering applications of molecular diagnostics in low-resource settings, e.g. developing countries.

Bacteria are a large group of unicellular microorganisms. The bacterial cell is surrounded by a cell membrane, which encloses the contents of the cell. In most bacteria, a peptidoglycan-based bacterial cell wall covers the outside of the cell membrane. It is located outside the cell membrane and provides the cell with structural support and protection, and also acts as a filtering mechanism.

Bacteria are often classified as Gram-negative or Gram-positive. Gram-staining is an empirical method of differentiating bacterial species based on the chemical and physical properties of their cells walls. Gram-negative bacteria typically have a thin cell wall consisting of only a few layers of peptidoglycan surrounded by a second lipid membrane containing lipopolysaccharides and lipoproteins. In contrast, Gram-positive bacteria typically possess thick mesh-like cell walls containing many layers of peptidoglycan and teichoic acids. For this reason many methods for bacterial cell lysis are specific either to Gram-negative or to Gram-positive bacteria; many methods that work well with Gram-negative bacteria do not work well with Gram-positive bacteria, and vice versa (see e.g. Salazar, Oriana, and Juan A. Asenjo. "Enzymatic lysis of microbial cells." Biotechnology letters 29.7 (2007): 985-994).

The differences between the requirements for lysing bacterial cells and for lysing eukaryotic cells are even larger (Shehadul Islam, Mohammed, Aditya Aryasomayajula, and Ponnambalam Ravi Selvaganapathy. "A Review on Macroscale and Microscale Cell Lysis Methods." Micromachines 8.3 (2017): 83). Animal cells are in general considered easier to lyse since they lack a cell wall. But also the composition of their plasma membranes is different from bacteria, for instance containing sterols, which increase the stability of the cells and makes them inflexible. Plant cells do have a cell wall; however, both its structure and function are completely different from bacterial cell walls. Plant cell walls consist of multiple layers which are primarily made up of cellulose, hemicellulose, pectin, lignin and structural proteins (Buchanan, Bob B., Wilhelm Gruissem, and Russell L. Jones. Biochemistry & molecular biology of plants. Vol. 40. Rockville, MD: American Society of Plant Physiologists, 2000). Bacterial cell walls on the other hand are made up of peptidoglycan. Also fungi have cell walls, which again differ from bacterial and plant cell walls in their makeup, consistng mainly of chitin, glucans and proteins (Webster, John, and Roland Weber. Introduction to fungi. Cambridge University Press, 2007).

Several methods for bacterial cell lysis are known in the art.

Mechanical methods include the use of a bead mill, disruption using a homogenizer, pressure using for example a French press, sonication, etc. These methods suffer from various disadvantages including high equipment costs and low throughput. Nonmechanical methods include thermal methods, such as the freeze/thaw method. Multiple cycles of freezing and thawing are necessary for efficient lysis, making the process lengthy. Enzymatic methods using for example lysozyme and proteases exist as well, however, such enzymes are usually not sufficiently effective by themselves and are only employed to make the lysis process in combination with another method more efficient.

In addition, cell lysis can be achieved by chemical methods, e.g. by changing the pH or by using detergents. Detergents are most widely used for lysing animal cells, which do not have a cell wall. For lysing bacterial cells, the cell wall has to be broken down in order to access the plasma membrane, which is why detergents can be used in combination with lysozyme. However, the detergents used are often not compatible with downstream applications such as NA amplification or sequencing and therefore require a purification step.

In recent years, novel approaches for the extraction of DNA from biological samples employed ionic liquids (ILs). ILs are salts that are liquid, e.g. at temperatures below 100 °C or even at room temperature. Depending on the nature of the cation and the anion ionic liquids can be miscible with water (hydrophilic) or immisicble with water (hydrophobic).

It has been shown that some ILs can be used to extract DNA from animal cells (Ressmann, Anna K., et al. "Fast and efficient extraction of DNA from meat and meat derived products using aqueous ionic liquid buffer systems." New Journal of Chemistry 39.6 (2015): 4994-5002) and from plant cells (Garcia, Eric Gonzalez, et al. "Direct extraction of genomic DNA from maize with aqueous ionic liquid buffer systems for applications in genetically modified organisms analysis." Analytical and Bioanalytical Chemistry 406.30 (2014): 7773-7784).

EP 2302030 A1 discloses specific types of ILs that can be used for the lysis of bacterial cells. A nucleic acid purification step using spin columns or magnetically attractable particles can be carried out after the lysis.

Fuchs-Telka et al. (Fuchs-Telka, Sabine, et al. "Hydrophobic ionic liquids for quantitative bacterial cell lysis with subsequent DNA quantification." Analytical and bioanalytical chemistry 409.6 (2017): 1503-1511) also discloses a method for bacterial cell lysis using specific ILs. However, the method only works for lysing Gram-negative bacterial cells, whereas "Gram-positive cells were protected by their thick cell wall."

EP 2702136 B1 discloses the use of water-immiscible ILs or oils for the lysis of bacterial cells. The disclosed method is described to work with Gram-negative bacteria; Gram-positive bacteria require a separate pre-incubation step and lysis at high temperatures (140 °C).

Despite currently available methods for lysing bacterial cells, new methods that address at least some of the disadvantages of existing methods are needed. It is an object of the present invention to provide such methods.

In the context of the present invention, it was surprisingly found that a lysis agent comprising a water-miscible ionic liquid containing choline can effectively lyse bacterial cells. Accordingly, the present invention relates to a method for lysing a bacterial cell comprising the steps of:
- providing a sample comprising the bacterial cell, and
- adding a lysis agent to create a lysis reaction mixture,
wherein the lysis agent comprises a water-miscible ionic liquid containing choline.

The inventive method can advantageously be used, for example, for extracting nucleic acids or other intracellular components from bacterial cells. Surprisingly, the inventive method is not limited to specific types of bacteria but can effectively be used for both Gram-negative and Gram-positive bacteria. The method according to the invention has a number of advantages over traditional methods of cell lysis. The procedure is very fast, easy to carry out and does not comprise many steps. The costs are low and no expensive equipment such as fume hoods are required. In addition, large amounts of waste and toxic or environmentally harmful chemicals can be avoided by using the inventive method.

In a further aspect the invention provides an apparatus for carrying out the inventive method, the apparatus comprising a container containing a lysis agent, wherein the lysis agent comprises a water-miscible ionic liquid containing choline.

A further aspect of the invention provides an apparatus for automated bacterial cell lysis comprising:
- a container containing a lysis agent,
- an automated liquid handling system for autonomously adding lysis agent to at least two, preferably at least 8, most preferably at least 96 samples comprising bacterial cells,
wherein the lysis agent comprises a water-miscible ionic liquid containing choline.

In a further aspect the invention provides a kit for NA amplification from a bacterial cell, the kit comprising:
- a lysis agent,
- a reagent for nucleic acid amplification, preferably selected from the group consisting of nucleoside triphosphates (NTPs), deoxynucleoside triphosphates (dNTPs), oligonucleotides, and NA amplification enzymes, preferably a DNA polymerase,
wherein the lysis agent comprises a water-miscible ionic liquid containing choline.

In a further aspect the invention provides a kit for NA isolation from a bacterial cell, the kit comprising:
- a lysis agent,
- a solid support for the adsorption of a NA, the solid support preferably being a spin column, a bead, or a microchip or channel,
wherein the lysis agent comprises a water-miscible ionic liquid containing choline.

ILs are salts which typically consist of an organic cation and an anion and have melting points typically below temperatures of 100 °C. Many ILs are even liquid at room temperature. Depending on the nature of the cation and the anion ionic liquids can be miscible with water (hydrophilic) or immiscible with water (hydrophobic; see Klähn, Marco, et al. "What determines the miscibility of ionic liquids with water? Identification of the underlying factors to enable a straightforward prediction." The Journal of Physical Chemistry B 114.8 (2010): 2856-2868, incorporated herein by reference).

The IL in the context of the invention is a water-miscible IL.

The term "water-miscible IL" as used in the context of the invention means that water and the IL can be mixed in all proportions, forming a homogeneous solution. In other words, water and the IL can fully dissolve in each other at any concentration.

Choline is a cation with the following chemical structure:

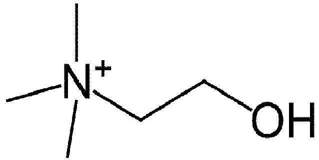

The IL containing the cation choline further contains an anion. In the course of the present invention it has been found that formate (Fmt), lactate (Lac), dibutylphosphate (DBP) and especially hexanoate (Hex) are particularly well suited anions. Accordingly, it is preferred if the IL further contains Fmt, Lac, DBP or Hex, most preferably Hex.

In a preferred embodiment the IL contains at least 2 % (w/w) (percent by weight) choline, preferably at least 5% (w/w), even more preferably at least 10% (w/w). In another preferred embodiment the IL contains at least 2% (w/w) Hex, preferably at last 5% (w/w), even more preferably at least 10% (w/w). It is especially preferred if choline and Hex in sum account for at least 50% (w/w), even more preferably at least 60% (w/w), yet even more preferably at least 70% (w/w), especially at least 80% (w/w), most preferably at least 90% (w/w) of the IL. In the context of the invention it is furthermore preferred, if the IL has a melting point below 90°C, more preferably below 70°C, even more preferably below 50°C, yet even more preferably below 40°C, especially below 30°C, most preferably below 20°C.

In a preferred embodiment of the invention, the method further further comprises the step of:
- heating the lysis reaction mixture to a temperature of at least 30°C, preferably at least 40°C, more preferably at least 50°C, even more preferably at least 60°C, most preferably at least 65°C.

It is however preferred if the lysis reaction mixture is not heated to a temperature higher than 100°C, preferably not higher than 90°C, more preferably not higher than 80°C, most preferably not higher than 70°C.

In another preferred embodiment, the inventive method further comprises the step of:
- incubating the lysis reaction mixture for at least 30 seconds, preferably at least 1 minute, more preferably at least 2 minutes, even more preferably at least 3 minutes, most preferably at least 5 minutes.

It is however preferred if the lysis reaction mixture is not incubated for more than 60 minutes, preferably not more than 30 minutes, more preferably not more than 20 minutes, even more preferably not more than 10 minutes.

It is especially preferred if during this time of incubation the lysis reaction mixture is kept at a temperature of at least 30°C, preferably at least 40°C, more preferably at least 50°C, even more preferably at least 60°C, most preferably at least 65°C, but preferably not higher than 100°C, more preferably not higher than 90°C, even more preferably not higher than 80°C, most preferably not higher than 70°C.

In a preferred embodiment of the invention the concentration of the ionic liquid in the lysis reaction mixture is at least 5% (w/v), preferably at least 10% (w/v), more preferably at least 20% (w/v), even more preferably at least 30% (w/v), most preferably at least 45% (w/v).

In further preferred embodiment, the lysis agent further comprises an aqueous buffer. It has been found in the course of the invention that Tris(hydroxymethyl)-aminomethan (Tris) and 2-(N-morpholino)ethanesulfonic acid (MES) are particularly well suited as buffer agents. In a preferred embodiment the lysis agent comprises at least 0.1 mM, preferably at least 1 mM Tris. In another preferred embodiment the lysis agent comprises at least 0.1 mM, perferably at least 1 mM MES. In all embodiments of the invention it is preferred if the lysis agent has a pH between 6 and 10, preferably between 7 and 9, more preferably between 7.5 and 8.5, most preferably 8.

The term "aqueous" as used herein means that the solvent comprises water. An "aqueous buffer" therefore refers to a solution containing a buffer agent such as Tris or MES dissolved in water. The term "aqueous" does, however, not exclude the presence of other solvents, such as a water-miscible organic solvent, e.g. an alcohol. As used herein, the term "aqueous" means that the concentration of water in a solution is at least 20% (w/v).

In yet another preferred embodiment, the concentration of the IL in the lysis agent is at least 10% (w/v), preferably at least 20% (w/v), more preferably at least 30% (w/v), even more preferably at least 40% (w/v), most preferably at least 50% (w/v).

In the course of the present invention it has surprisingly been found that the inventive method is suitable for use with both Gram-positive and with Gram-negative cells. Accordingly in a preferred embodiment of the invention the bacterial cell is a Gram positive bacterial cell. In another preferred embodiment the bacterial cell is a Gram negative bacterial cell.

The method according to the invention is particularly well suited for extracting a NA form a bacterial cell. The NA, preferably DNA or RNA, can further be processed e.g. in a NA amplification reaction. To avoid inhibition or interference with such an amplification reaction, it is preferred if the lysis reaction mixture is diluted prior to said reaction.

Accordingly, in another embodiment of the invention the method further comprises the steps of
- diluting the lysis reaction mixture with an aqueous solution,
- using the lysis reaction mixture in a nucleic acid amplification process, preferably in a PCR, most preferably in a qPCR.

In the context of this embodiment, it is preferred if the aqueous solution is an aqueous buffer, preferably Tris or MES buffer.

In the context of the invention, the nucleic acid amplification process can be any method for amplifying NAs, preferably DNA or RNA. Preferably the NA amplification process is a polymerase chain reaction (PCR), especially a quantitative polymerase chain reaction (qPCR) or real-time PCR. In another embodiment the NA amplifcation process is an isothermal amplification reaction, such as a loop-mediated isothermal amplification (LAMP), a strand displacement amplification (SDA), a helicase-dependent amplification (HDA) or a nicking enzyme amplification reaction (NEAR). In yet another embodiment, the NA amplification process is a NA sequencing process, preferably a DNA sequencing process.

NA amplification processes such as qPCR are widely used in microbial diagnostics (see Kralik, Petr, and Matteo Ricchi. "A basic guide to real time PCR in microbial diagnostics: Definitions, parameters, and everything." Frontiers in microbiology 8 (2017): 108, incorporated herein by reference). Accordingly, in a preferred embodiment, the method further comprises the step of: - detecting the presence of a specific type of bacterium in the sample.

In the context of the entire invention, the sample preferably is a food sample, a drinking water sample, an environmental sample such as a soil or water sample, or an animal sample, preferably a human sample. When the sample is an animal sample, e.g. a human sample, it especially preferred if the sample is a stool sample or a blood sample. In a preferred embodiment the specific type of bacterium is a pathogenic bacterium.

In a preferred embodiment, the lysis reaction mixture is diluted with an aqueous solution by a factor of at least 1 (i.e. equal volume of lysis reaction mixture and aqueous solution mixed), preferably by a factor of at least 2, more preferably by a factor of at least 5, even more preferably by a factor of at least 10, most preferably by a factor of at least 20 (i.e. 1 part lysis reaction mixture mixed with 19 parts aqueous solution).

It is known in the art that a downside of most hydrophilic ILs is interference with or even complete inhibition of subsequent molecular biological methods, such as qPCR, and that they usually must be removed before analysis (Fuchs-Telka, Sabine, et al. "Hydrophobic ionic liquids for quantitative bacterial cell lysis with subsequent DNA quantification." Analytical and bioanalytical chemistry 409.6 (2017): 1503-1511). In the course of the present invention it was surprisingly found that the lysis reaction mixture can be used in PCR reactions such as qPCR without prior removal of the IL. This is very convenient since purification steps can be avoided.

Accordingly, in a preferred embodiment of the invention, the IL is not removed from the lysis reaction mixture before the NA amplification process.

In an especially preferred embodiment, the lysis reaction mixture is not subjected to any purification steps.

The inventive method can also advantageously be used for the purification of nucleic acids from a bacterial cell. Accordingly in a further preferred embodiment of the invention the method further comprises the step of
- purifying a nucleic acid, preferably DNA, most preferably genomic DNA, from the lysis reaction mixture, preferably by adsorption to a silica surface, preferably of a spin column.

Many methods for purifying nucleic acids are known in the art and can be used in the context of the invention, e.g. using spin columns, magnetic particles, microchips or phenol precipitation. Spin columns and magnetic particles for NA purification are widely commercially available. All such methods can suitably be used in the context of the present invention.

All detailed descriptions of the inventive method, e.g. related to the lysis agents or ionic liquids, also apply to the apparatus and to the kits according to the invention.

Automated liquid handling systems are commonly used in chemical or biochemical laboratories. A large number of automated liquid handling systems are commercially available. Examples include QIAgility (Qiagen), epMotion (Eppendorf), Fluent (Tecan), and Freedom EVO (Tecan). Typically such automated liquid handling systems comprise a motorized pipette or syringe attached to a robotic arm. In this way, such systems are typically able to dispense selected quantities of liquids to designated containers. Such liquid handling systems can contain further features e.g. for heating and/or mixing samples. Automated liquid handling systems are advantageously used for processing multiple samples automatically, e.g. in 96-well plates.

It is preferred if the apparatus according to the invention comprises an automated liquid handling system, preferably a motorized pipette or syringe, preferably attached to a robotic arm. It is furthermore preferred, if the apparatus comprises a heating system, preferably a heating block. In this way, the apparatus can carry out the method according to the invention with multiple samples autonomously, i.e. without intervention by the user.

In a preferred embodiment, the apparatus is adapted to process multiple samples in parallel. "In parallel" in this context does not necessarily mean that all steps of the inventive method have to happen with each of the samples at exactly the same time. It rather means that the user can provide multiple samples to the apparatus and the apparatus can autonomously process these multiple samples in a certain period of time without the user having to intervene, e.g. by switching samples. It is especially preferred if the apparatus is adapted to process at least 8 samples, more preferably at least 24 samples, most preferably at least 96 samples in parallel.

Commonly multiwell plates such as 96-well plates are used for processing multiple samples in parallel. In a preferred embodiment, the apparatus is therefore adapted to process samples contained in such plates. It is especially advantageous if the bacterial cells are cultured directly in such plates. The multiwell plates can then simply be provided to the apparatus, without the user having to carry out any liquid transfer steps.

In a preferred embodiment, the apparatus is furthermore adapted to autonomously carry out NA purification or NA amplification from the sample. NA purification robots are commonly used in biochemical laboratories, e.g. the commercially available QI-Acube HT (Qiagen). Such robots typically contain a vacuum pump for drawing samples and reagents through columns containing a solid support, e.g. a silica membrane. In a preferred embodiment the apparatus therefore further contains a vacuum pump.

An apparatus according to the invention can be used for lysing multiple samples of bacterial cells automatically. For example, the user can provide multiple samples of bacterial cells, e.g. in a 96-well plate, to the apparatus. The apparatus then transfers a certain volume of lysis agent to each sample and preferably mixes the sample and the lysis agent, e.g. by shaking or by pipetting up and down repeatedly. The apparatus preferably incubates the lysis reaction mixture at a temperature and for a period of time according to the inventive method using an integrated heating system. Optionally, the apparatus can then transfer a certain volume of aqueous buffer from a second container to each sample or carry out a NA purification step, e.g. using columns or magnetic beads containing a solid support for the adsorption of NAs, e.g. containing a silica surface. All of these steps can happen autonomously, i.e. without intervention by the user. The samples are then ready to be used for further applications, e.g. a NA amplification reaction. Any kit according to the invention preferably comprises one or multiple items selected from the group consisting of instructions for use, Eppendorf tubes or other containers, buffers, and controls.

The inventive kit for NA amplification from a bacterial cell can be used in method involving a NA amplification process, preferably involving PCR, most preferably qPCR. The inventive kit can in this way be used for detecting a specific type bacterium in a sample. Accordingly, the present invention also relates to the use of the inventive kit for detecting a specific type of bacterium in a sample. The sample preferably is a food sample, a drinking water sample, an environmental sample such as a soil or water sample, or an animal sample, preferably a human sample. When the sample is an animal sample, e.g. a human sample, it especially preferred if the sample is a stool sample or a blood sample. The specific type of bacterium preferably is a pathogenic bacterium. It is especially preferred if the inventive kit is used for amplifying a NA from a bacterial cell using a method according to the invention.

The inventive kit for NA isolation from a bacterial cell can be used in a method involving a NA isolation process. Accordingly, the present invention also relates to the use of the inventive kit for isolating a NA, preferably DNA, more preferably genomic DNA, from a bacterial cell.

The inventive method and kits find their use in many different applications, e.g. in clinical microbiology, food and water hygiene, environmental hygiene or microbiological and pharmaceutical research.

Percentages (%) as used herein correspond to weight per volume (w/v) unless specified as weight per weight (w/w) or otherwise.

The present invention is further illustrated by the following figures and examples, without being limited thereto.

### Figures

**Figure 1****.** Results of the qPCR analysis of eight ionic liquids in different concentrations spiked with a DNA plasmid standard (10⁴ DNA target copies in each reaction). The ILs were diluted using (A) Tris buffer, (B) MES buffer, and (C) sodium phosphate buffer. The whiskers indicate the standard deviations of the qPCR triplicates.
**Figure 2****.** *Enterococcus* 23S rRNA gene copy number (log10-transformed) measured by qPCR after cell lysis experiments with eight different ILs (90% (w/v)) diluted with Tris or MES buffer.
**Figure 3****.** *Enterococcus* 23S rRNA gene copy number (log10-transformed) measured by qPCR after cell lysis experiments with varying concentrations of [C2mim]OAc and [Cho]Hex. The extraction variants were carried out in five replicates each.
**Figure 4****.** *Enterococcus* 23S rRNA gene copy number (log10-transformed) measured by qPCR after a five-fold replication of the cell lysis experiments submitted to varying temperatures and incubation periods.
**Figure 5****.** Number of detected 16S rRNA gene copies in the DNA extracts obtained from five extraction methods applied to (A) four Gram-positive, and (B) four Gram-negative bacterial reference strains. The extractions were carried out in triplicate for each strain and extraction method.

### Example 1. Materials and methods

### Ionic Liquids

In total, eight ionic liquids were used in the context of the following examples:

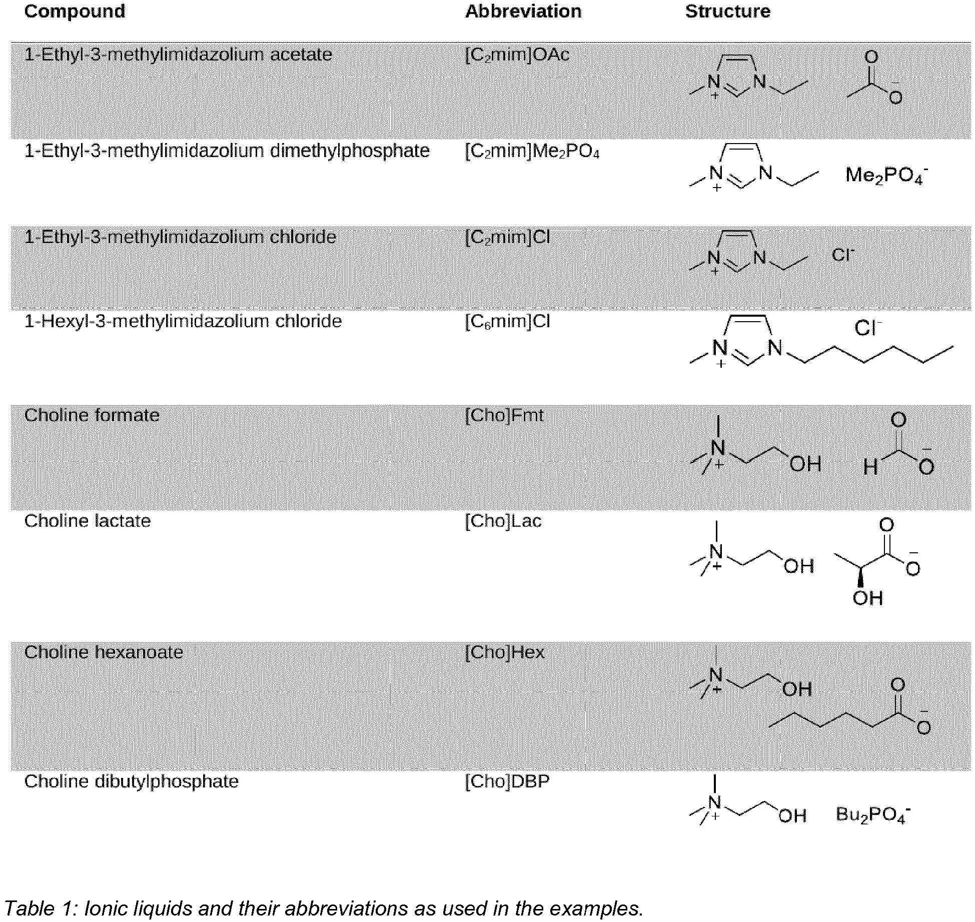

Commercially available reagents and solvents for the synthesis of ionic liquids were used as received from Sigma Aldrich unless otherwise specified. 1-Ethyl-3-methylimidazolium acetate, ([C2mim]OAc), 1-ethyl-3-methylimidazolium chloride ([C2mim]Cl), and choline dibutyl phosphate were purchased from Iolitec (Heilbronn, Germany) and used as received.

Choline based ionic liquids [Cho]Fmt, [Cho]Lac and [Cho]Hex, were prepared according to literature procedures, relying on the neutralization of freshly titrated commercially available choline bicarbonate solution with the corresponding acid in a ratio 1:0.95 to avoid the presence of any excess acid as exemplified on the synthesis of choline hexanoate:
A freshly titrated solution of choline bicarbonate (19.55 g, 90.80 mmol) was charged into a 3-necked round bottom flask and it was diluted with distilled water. Hexanoic acid (10.02 g, 86.26 mmol) was added dropwise to the reaction mixture. The reaction mixture was stirred at room temperature and concentrated in vacuo. Remaining solvent traces were removed under vacuum (0.2 mbar) with stirring for 20 hours at 40 °C. The product was obtained as light yellowish gel (20.94 g, >99% yield). 1H NMR (400 MHz, CDCl3) δ = 3.94 (s, 2H, CH2-OH), 3.57 (t, J = 4.42 Hz, 2H, CH2-CH2-OH), 3.25 (s, 9H, 3 x CH3), 2.00 (t, J = 6.76 Hz, 2H, CH2-COO), 1.46 (quin., J = 8.05 Hz, 2H, CH2-CH2-COO), 1.18 (m, 4H, CH2-(CH2)2-COO, CH2-(CH2)3-COO), 0.77 (t, J = 6.76 Hz, 3H, CH3-(CH2)4-COO). 13C NMR (100 MHz, D2O) δ = 183.95 (1C, COO), 67.35 (1C, CH2-OH), 55.52 (1C, CH2-CH2-OH), 53.92 (3C, 3 x CH3), 37.58 (1C, CH2-COO), 30.99 (1C, CH2-CH2-COO), 25.55 (1C, CH2-(CH2)2-COO), 21.76 (1C, CH2-(CH2)3-COO), 13.29 (1C, CH3-(CH2)4-COO).

### Bacterial Strains

Pure cultures of a total of eight bacterial type strains were used for the DNA extraction experiments of Examples 3 to 6. Of these eight strains, four belong to the group of Gram-positive bacteria (*Enterococcus faecalis* NCTC 775, *Clostridium perfringens* NCTC 8237, *Bacillus subtilis* ATCC 6633, *Staphylococcus aureus* NCTC 6571) and four to the group of Gram-negative bacteria (*Escherichia coli* NCTC 9001, *Legionella pneumophila* NCTC 12821, *Pseudomonas aeruginosa* NCTC 10662, *Vibrio cholerae* ATCC 51352). For the screening experiments with the ionic liquids of Examples 3 to 5, *Enterococcus faecalis* NCTC 775 was cultivated at 37 °C in tryptic soy broth with yeast extract. The harvested liquid cultures were stored in 25% (w/v) glycerol on - 80 °C until further use. For the comparison of the five extraction methods in Example 6, the cells of all eight strains were grown on agar plates and suspended in Ringer's solution for the successive cell count and extraction experiments. The cell suspensions were stored in 25% (w/v) glycerol on -80 °C until further use.

### Total cell count by flow cytometry and fluorescence microscopy

Dilutions of the bacterial cell suspensions were mixed with fluorescein isothiocyanate solution and incubated at 37 °C for 15 minutes. The samples were subsequently analysed for their total cell count using an Attune NxT flow cytometer (Life Technologies, Darmstadt, Germany) equipped with a 488 nm flat-top laser at 50 mW. In parallel, cell aliquots were fixed with paraformaldehyde, filtered, and stained with SYBR Gold for a subsequent total cell count under a fluorescence microscope.

### Quantification of bacterial DNA using quantitative PCR Enterococcus-specific qPCR assay

To quantify the DNA in the inhibition and cell lysis experiments of Examples 2 to 5 for which we used *Enterococcus faecalis* as model organism, we applied a qPCR assay that specifically targets a region in the *Enterococcus* 23S rRNA gene (ENT-qPCR) ("Method 1611: Enterococci in Water by TaqMan® Quantitative Polymerase Chain Reaction (qPCR) Assay." *US Environmental Protection Agency, Washington, DC* (2012)). The qPCR reactions were carried out in a total reaction volume of 15 µl containing 1 µM of each primer (MWG-Biotech AG, Ebersberg, Germany), 80 nm of the probe (all oligonucleotide sequences are listed in Table 3), KAPATM Probe® Fast qPCR Master Mix 2X (Peqlab, Erlangen, Germany), and 2.5 µl DNA extract. The reactions were performed on a 7500 Fast Real-Time PCR System (Applied Biosystems, New York, USA) according to the following protocol: 5 min at 95 °C, followed by 45 cycles of 15 s at 95 °C and 1 min at 60 °C. Unless noted otherwise, qPCR reactions were carried out in triplicate. The calibration curve was generated using a dilution series of DNA plasmid solution containing the 23S rRNA gene fragment that is targeted by the assay.

### Bacteria-specific qPCR assay

To quantify the DNA in the extraction experiment of Example 6 using eight different bacterial strains, we applied a qPCR assay that targets the V1-V2 region of the 16S rRNA gene that is universal to all bacteria (16S-qPCR) (Savio, Domenico, et al. "Bacterial diversity along a 2600 km river continuum." Environmental microbiology 17.12 (2015): 4994-5007). The qPCR reactions were carried out in a total reaction volume of 15 µl containing 200 nM of each primer (MWG-Biotech AG, Ebersberg, Germany; oligonucleotide sequences are listed in Table Tabelle), 12.5 µl KAPATM SYBR® Fast qPCR Master Mix 2X (Peqlab, Erlangen, Germany), 0.4 µg/µl BSA, and 2.5 µl DNA extract. The reactions were performed on a 7500 Fast Real-Time PCR System (Applied Biosystems, New York, USA) according to the following protocol: 3 min at 95 °C, followed by 40 cycles of 30 s at 95 °C, 30 s at 57 °C, 1 min at 72 °C, and a final elongation step for 2 min at 72 °C. Unless noted otherwise, qPCR reactions were carried out in duplicate. The calibration curve was generated using a dilution series of DNA plasmid solution containing the 16S rRNA gene fragment that is targeted by the assay.

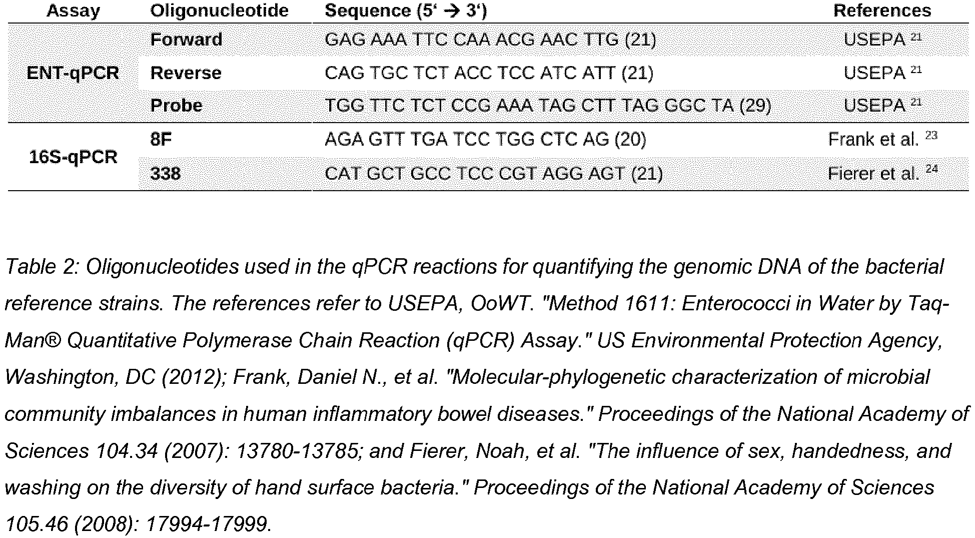

### Example 2. Influence of ionic liquids and buffer systems on qPCR.

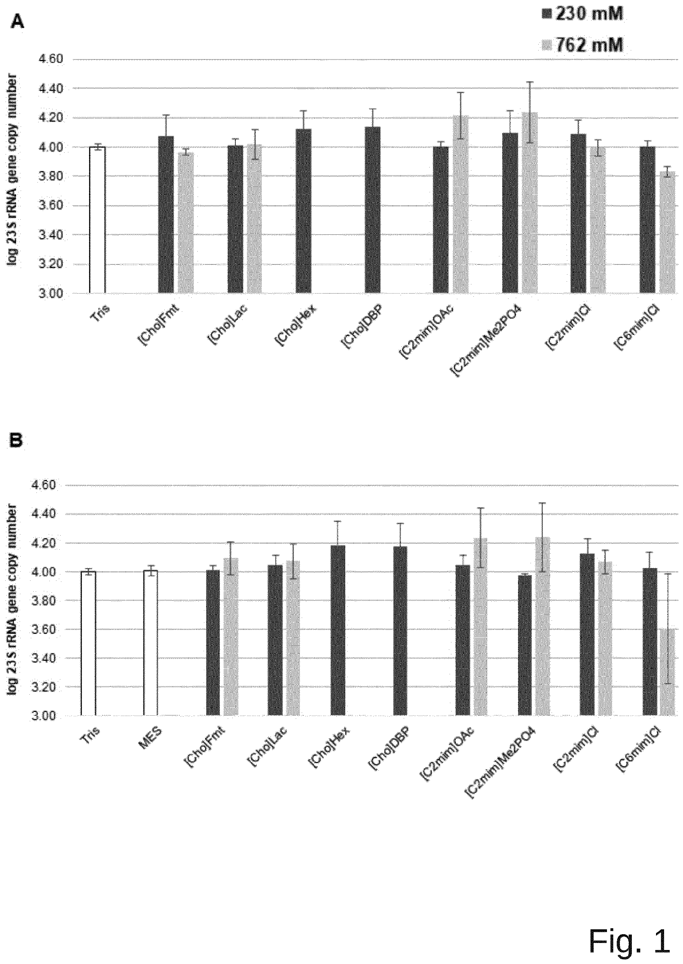

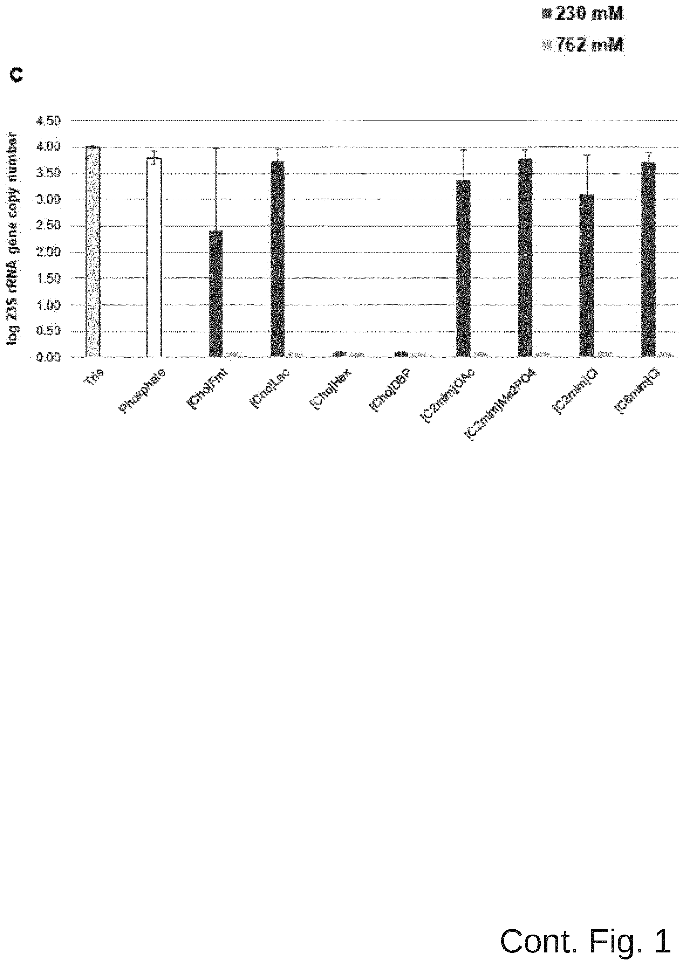

In a set of experiments, we initially determined the tolerable concentration of the selected ILs for the use in quantitative PCR experiments by testing for inhibitory effects on the amplification reaction. For the dilution of these ILs, we used three buffer systems, namely Tris(hydroxymethyl)aminomethane (Tris, 10 mM, pH 8.0); 2-(N-morpholino)ethanesulfonic acid (MES, 50 mM, pH 6.0); and sodium phosphate (50 mM, pH 8.5). Subsequently, we spiked four different concentrations of the ILs (230 mM; 762 mM; 1250 mM; 2300 mM) with a DNA plasmid standard containing 10⁴ copies of a diagnostic fragment of the *Enterococcus* spp. 23S rRNA gene. The DNA standard variants were then analysed by applying the *Enterococcus-specific* qPCR assay reported in Example 1 (ENT-qPCR; Figure 1). The best results for all ILs were obtained with 230 mM ILs diluted with Tris and MES buffer where no inhibitory effects on the qPCR reaction occurred. At concentrations of 762 mM, [Cho]Hex and [Cho]DBP already completely inhibited the amplification, and [C6mim]Cl substantially interfered with the reaction. In contrast, the sodium phosphate buffer system interfered with the qPCR reactions already without ILs and partially or completely inhibited the amplification reaction at IL concentrations of 230 mM and 762 mM (Figure 1, C).

### Example 3. Cell lysis experiments.

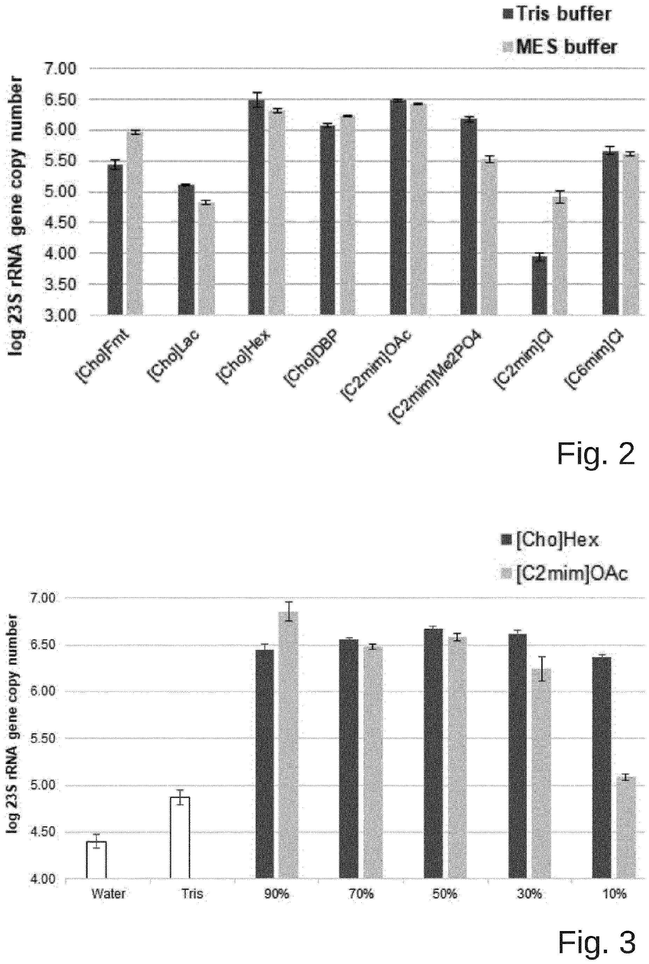

In a next step, we tested the effect of the ILs on the lysis of Gram-positive bacterial cells. For this purpose, we used En*terococcus faecalis* type strain NCTC 775 as model organism for Gram-positive bacteria. *Enterococcus* species are ubiquitous in nature and hold important relevance in clinical, food, and environmental diagnostics. Furthermore, the ENT-qPCR assay as disclosed in Example 1 represents a reliable method that is used by the U.S. EPA for the routine monitoring of bathing water quality. First, we cultivated the cells in liquid media and counted the cells with flow cytometry and fluorescence microscopy as disclosed in Example 1 at different time points to determine the optical cell density at 670 nm (OD670) and the corresponding cell number. For the cell lysis experiments, we harvested the cells after five hours at OD670=0.2, corresponding to approximately 10⁸ cells per ml. This approach ensured a reproducible condition of an early growth phase where most of the cells were dividing and the percentage of dead cells was at a low level. To remove potentially dead cells and free DNA as well as nutrient medium and glycerol from the liquid culture, the cells were pelleted, washed, and resuspended in the same buffer system that we subsequently used for the cell lysis experiments (Tris and MES). We mixed 10 µl each of resuspended washed cells with 90 µl each of the ionic liquids at a concentration of 90% (w/v). For a first screening, we incubated these reaction mixtures for 30 minutes at 95 °C with short vortexing steps every 10 minutes. To avoid PCR inhibition due to the high IL concentrations, we diluted the crude extracts with the corresponding buffer in a 1:20 ratio and applied the ENT-qPCR assay to quantify the released DNA target molecules (Figure 2). We detected approximately log 6.49 ± 0.11 and log 6.48 ± 0.02 23S rRNA gene copies in 2.5 µl of the DNA extracts that resulted from the two best performing ionic liquids, [Cho]Hex and [C2mim]OAc. The DNA yields obtained with these ILs in MES buffer were slightly lower than with the Tris buffer system.

### Example 4. IL concentration.

To investigate the influence of varying IL concentrations on the cell lysis efficiency, we applied the selected ILs ranging from 90% (w/v) to 10% (w/v) to the same extraction procedure as previously described. In addition, we only added double-distilled water or Tris buffer to the cells in order to investigate the difference in the relative cell lysis efficiency with and without the respective ILs (Figure 3). The efficiency of [C2mim]OAc almost steadily decreased with its respective concentration, while the performance of [Cho]Hex slightly improved towards a concentration of 50% (w/v), only decreasing with lower concentrations of 30% (w/v) and 10% (w/v), respectively. As expected, heating the cell suspension with double-distilled water or Tris buffer resulted in a significantly lower yield of extracted DNA. Due to the high yields obtained with the respective IL concentrations, we selected 90% (w/v) [C2mim]OAc and 50% (w/v) [Cho]Hex for all subsequent experiments.

### Example 5. Extraction conditions

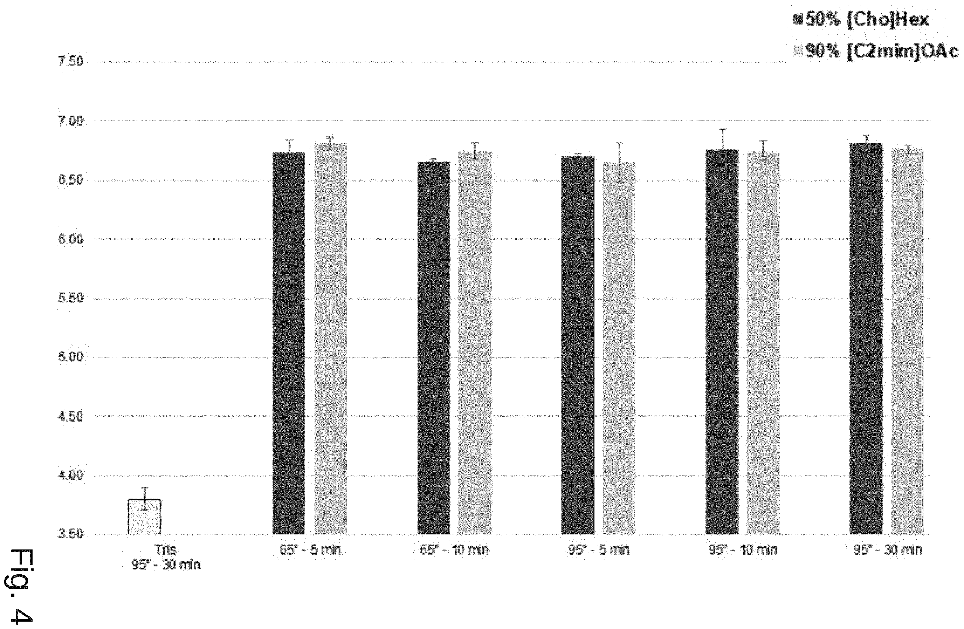

To further optimize the extraction procedure, we incubated the E. faecalis cells with the selected ILs at 95 °C and 65 °C for three different time periods in five replicates each. Figure 4 shows that no significant differences between the variations occurred, therefore an incubation time and temperature of 5 min at 65 °C was selected for the further experiments disclosed in Example 6.

### Example 6. Comparison of the inventive method with conventional methods based on eight bacterial reference strains

Finally, we assessed the performance of the IL-based DNA extraction methods in comparison with a DNA extraction method based on enzymatic lysis with phenol/chloroform purification (Phe/Chl), and two commercial kits. For this purpose, we cultivated a set of four Gram-positive and four Gram-negative bacterial species, extracted them with both ionic liquids as well as the three selected procedures and performed a subsequent qPCR analysis of the extracts. To represent Gram-positive bacteria, we selected *Clostridium perfringens, Bacillus subtilis,* and *Staphylococcus aureus* in addition to *Enterococcus faecalis.* Furthermore, we tested the ILs also on Gram-negative species for which we selected *Escherichia coli, Legionella pneumophila, Pseudomonas aeruginosa,* and *Vibrio cholerae* as model organisms. Some of these strains are commonly used as indicator bacteria, whereas others represent widespread human pathogens that are clinically relevant or are attributed to food spoilage, respectively. To avoid the use of eight different species-specific qPCR assays while ensuring the comparability of the results, we analysed all DNA extracts with a qPCR assay targeting a 16S rRNA gene fragment that is universal to all bacteria (see Example 1).

For preparing bacterial suspensions for the subsequent extraction experiments, aliquots of the liquid cultures or the suspensions were centrifuged at 10,000 rpm, and the resulting cell pellets were washed twice and resuspended in the respective buffer that was used for the ionic liquid dilutions. Ten µl of the respective cell suspensions were used for each extraction procedure. The following extraction procedures were used:
Optimized extraction procedure using IL/aqueous buffer systems: Ten µl of the pelleted and resuspended cells were mixed with 90 µl of the respective IL/buffer system (90% (w/v) [C2mim]OAc or 50% (w/v) [Cho]Hex) and incubated at 65 °C for 5 min. To overcome inhibitory effects caused by the ILs or cell components, we diluted the extract with 10 mM Tris pH 8.0 in a 1:20 ratio for the subsequent qPCR analyses.

Extraction procedure using lysozyme/proteinase K with phenol/chloroform purification: Briefly, 10 µl of each cell suspension in TE buffer were incubated twice for one hour at 37 °C after the additions of lysozyme and proteinase K, respectively. After another 10 min incubation with added sodium chloride and CTAB, the released DNA was thereafter separated from other cell components by the treatment with a combination of phenol and chloroform/isoamylalcohol. Finally, the DNA was precipitated using isopropanol, followed by a washing step with ethanol and the addition of 10 mM Tris pH 8.0 for resuspending the DNA pellet.

Extraction procedure using commercial kits: For the comparison of the extraction efficiencies with commercial kits, we used the PeqGOLD Bacterial DNA Mini Kit, the QIAamp DNA Mini Kit from Qiagen and the Wizard Genomic DNA Purification Kit from Promega. The extraction procedures were carried out according to the manufacturers' instructions for Gram-positive and Gram-negative bacteria using 10 µl of the respective cell suspensions.

All DNA extracts were analyzed with a qPCR assay targeting a 16S rRNA gene fragment as described in Example 1. Since the bacterial suspensions only contained approximate numbers of cells that were potentially reduced during the process of pelleting and washing, the results for the different strains cannot not be quantitatively compared to each other directly.

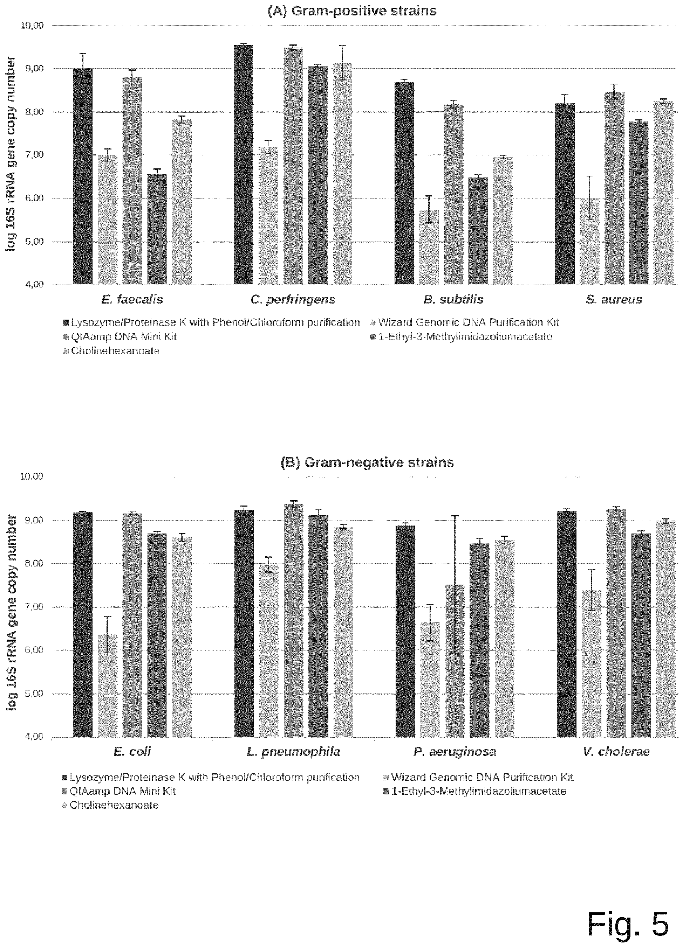

As can be seen from the results presented in Figure 5, the inventive method using ILs could successfully used for all the strains tested. Importantly, the extraction methods using the ILs gave even better results than the commercial Wizard Genomic DNA Purification Kit from Promega.

### Example 7. Costs and duration of the inventive method compared with conventional methods

Since the commercial kits come in several sizes and therefore vary in relative prices, we considered the lowest possible price per sample for the following calculations. For the enzymatic extraction, we assumed that the necessary reagents are acquired in reasonable amounts for small to medium-sized labs, thus calculating an average price for chemicals such as phenol, chloroform, or ethanol. Although [Cho]Hex is not in the assortment of common commercial suppliers, it can be custom-synthesized by companies such as Iolitec (Heilbronn, Germany), starting from 50 g batches. Since we considered this minimum orderable amount for the calculations, it can be assumed that the price per extraction with [Cho]Hex can be further decreased when ordering larger quantities. On the other hand, [C2mim]OAc is commercially available (e.g., Merck) in different amounts, for which we also assumed the lowest possible price per sample.

**Table 3: Overview on approximate prices and durations per sample, calculated for the five extraction methods that were used in this study. The prices also include the costs for pipette tips and reaction tubes but neglect the personnel costs that arise from the working hours. The durations reflect the sum of all incubation and centrifugation steps, but do not include buffer preparation and general handling, such as pipetting, centrifuge (un)loading, or reaction tube labelling.**

| **Extraction method** | **Price per sample** | **Duration per sample** |
|---|---|---|
| [Cho]Hex | 0.73€ | 5 min (Gram+ and -) |
| [C2mim]OAc | 1.14€ | 5 min (Gram+ and -) |
| Phe/Chl | 1.46€ | 180 min (Gram+) |
| | | 120 min (Gram-) |
| Promega | 2.31€ | 134-214 min (Gram+) |
| | | 102-152 min (Gram-) |
| Qiagen | 3.10€ | 87 min (Gram+) |
| | | 22 min (Gram -) |

In times of massive environmental pollution from toxins and plastic waste, one must also consider the use of volatile halogenated organic solvents in traditional enzymatic methods, as well as the excessive packaging of consumables that comes with some commercial kits. In contrast, the extraction with ionic liquids can be carried out in a single tube and thereby offers a low environmental footprint, especially in combination with the use of biodegradable molecules such as choline hexanoate.

The present invention relates to the following preferred embodiments:
1. Method for lysing a bacterial cell comprising the steps of:
   - providing a sample comprising the bacterial cell, and
   - adding a lysis agent to create a lysis reaction mixture, wherein the lysis agent comprises a water-miscible ionic liquid containing choline.
2. Method according to embodiment 1, wherein the ionic liquid further contains formate (Fmt), lactate (Lac), dibutylphosphate (DBP) or hexanoate (Hex), most preferably Hex.
3. Method according to embodiment 1 or 2, further comprising the step of:
   - heating the lysis reaction mixture to a temperature of at least 30°C, preferably at least 40°C, more preferably at least 50°C, even more preferably at least 60°C, most preferably at least 65°C.
4. Method according to any one of embodiments 1 to 3, wherein the lysis reaction mixture is not heated to a temperature higher than 100°C, preferably not higher than 90°C, more preferably not higher than 80°C, most preferably not higher than 70°C.
5. Method according to any one of embodiments 1 to 4, further comprising the step of:
   - incubating the lysis reaction mixture for at least 30 seconds, preferably at least 1 minute, more preferably at least 2 minutes, even more preferably at least 3 minutes, most preferably at least 5 minutes.
6. Method according to any one of embodiments 1 to 5, wherein the lysis reaction mixture is not incubated for more than 60 minutes, preferably not more than 30 minutes, more preferably not more than 20 minutes, even more preferably not more than 10 minutes.
7. Method according to any one of embodiments 1 to 6, wherein the concentration of the ionic liquid in the lysis reaction mixture is at least 5% (w/v), preferably at least 10% (w/v), more preferably at least 20% (w/v), even more preferably at least 30% (w/v), most preferably at least 45% (w/v).
8. Method according to any one of embodiments 1 to 7, wherein the lysis agent further comprises an aqueous buffer.
9. Method according to any one of embodiments 1 to 8, wherein the bacterial cell is a Gram positive bacterial cell.
10. Method according to any one of embodiments 1 to 9, wherein the bacterial cell is a Gram negative bacterial cell.
11. Method according to any one of embodiments 1 to 10, further comprising the steps of
   - diluting the lysis reaction mixture with an aqueous solution,
   - using the lysis reaction mixture in a nucleic acid amplification process, preferably in a PCR, most preferably in a qPCR.
12. Method according to any one of embodiments 1 to 11, further comprising the step of
   - purifying a nucleic acid, preferably DNA, most preferably genomic DNA, from the lysis reaction mixture, preferably by adsorption to a silica surface, preferably of a spin column.
13. Apparatus for carrying out the method of any one of embodiments 1 to 12, the apparatus comprising a container containing a lysis agent, wherein the lysis agent comprises a water-miscible ionic liquid containing choline.
14. Apparatus according to embodiment 13, further comprising an automated liquid handling system.
15. Apparatus for automated bacterial cell lysis comprising:
   - a container containing a lysis agent,
   - an automated liquid handling system for autonomously adding lysis agent to at least two, preferably at least 8, most preferably at least 96 samples comprising bacterial cells,
   wherein the lysis agent comprises a water-miscible ionic liquid containing choline.
16. Apparatus according to any one of embodiments 13 to 15, wherein the automated liquid handling system is or comprises a motorized pipette or syringe, preferably attached to a robotic arm.
17. Apparatus according to any one of embodiments 13 to 16, wherein the ionic liquid further contains formate (Fmt), lactate (Lac), dibutylphosphate (DBP) or hexanoate (Hex), most preferably Hex.
18. Apparatus according to any one of embodiments 13 to 17, wherein the concentration of the ionic liquid in the lysis reaction mixture is at least 5% (w/v), preferably at least 10% (w/v), more preferably at least 20% (w/v), even more preferably at least 30% (w/v), most preferably at least 45% (w/v).
19. Apparatus according to any one of embodiments 13 to 18, further comprising a second container containing an aqueous buffer.
20. Apparatus according to any one of embodiments 13 to 19, further comprising a heating system, preferably a heating block.
21. Apparatus according to any one of embodiments 13 to 20, wherein the apparatus is adapted to process multiple samples, preferably at least 8 samples, more preferably at least 96 samples, in parallel.
22. Apparatus according to any one of embodiments 13 to 21, wherein the apparatus is adapted to process samples contained in a multiwell plate, preferably a 96-well plate.
23. Apparatus according to any one of embodiments 13 to 22, wherein the apparatus comprises a vacuum pump.
24. A kit for NA amplification from a bacterial cell, the kit comprising:
   - a lysis agent,
   - a reagent for nucleic acid amplification, preferably selected from the group consisting of nucleoside triphosphates (NTPs), deoxynucleoside triphosphates (dNTPs), oligonucleotides, and NA amplification enzymes, preferably a DNA polymerase,
   wherein the lysis agent comprises a water-miscible ionic liquid containing choline.
25. A kit for NA isolation from a bacterial cell, the kit comprising:
   - a lysis agent,
   - a solid support for the adsorption of a NA, the solid support preferably being a spin column, a bead, or a microchip or channel,
   wherein the lysis agent comprises a water-miscible ionic liquid containing choline.
26. Kit according to embodiment 24 or 25, wherein the ionic liquid further contains formate (Fmt), lactate (Lac), dibutylphosphate (DBP) or hexanoate (Hex), most preferably Hex.
27. Kit according to any one of embodiments 24 to 26, wherein the concentration of the ionic liquid in the lysis reaction mixture is at least 5% (w/v), preferably at least 10% (w/v), more preferably at least 20% (w/v), even more preferably at least 30% (w/v), most preferably at least 45% (w/v).
28. Kit according to any one of embodiments 24 to 27, wherein the kit comprises an apparatus according to any one of embodiments 13 to 23.

## Claims

1. Method for lysing a bacterial cell comprising the steps of:
- providing a sample comprising the bacterial cell, and
- adding a lysis agent to create a lysis reaction mixture, wherein the lysis agent comprises a water-miscible ionic liquid containing choline.

2. Method according to claim 1, wherein the ionic liquid further contains formate (Fmt), lactate (Lac), dibutylphosphate (DBP) or hexanoate (Hex), most preferably Hex.

3. Method according to claim 1 or 2, further comprising the step of:
- heating the lysis reaction mixture to a temperature of at least 30°C, but preferably not higher than 100°C.

4. Method according to any one of claims 1 to 3, further comprising the step of:
- incubating the lysis reaction mixture for at least 30 seconds, but preferably not more than 60 minutes.

5. Method according to any one of claims 1 to 4, wherein the concentration of the ionic liquid in the lysis reaction mixture is at least 5% (w/v), preferably at least 10% (w/v), more preferably at least 20% (w/v), even more preferably at least 30% (w/v), most preferably at least 45% (w/v).

6. Method according to any one of claims 1 to 5, wherein the bacterial cell is a Gram positive bacterial cell.

7. Method according to any one of claims 1 to 6, wherein the bacterial cell is a Gram negative bacterial cell.

8. Method according to any one of claims 1 to 7, further comprising the steps of
- diluting the lysis reaction mixture with an aqueous solution,
- using the lysis reaction mixture in a nucleic acid amplification process, preferably in a PCR, most preferably in a qPCR.

9. Method according to any one of claims 1 to 8, further comprising the step of
- purifying a nucleic acid, preferably DNA, most preferably genomic DNA, from the lysis reaction mixture, preferably by adsorption to a silica surface, preferably of a spin column.

10. Apparatus for carrying out the method of any one of claims 1 to 9, the apparatus comprising a container containing a lysis agent, wherein the lysis agent comprises a water-miscible ionic liquid containing choline.

11. Apparatus for automated bacterial cell lysis comprising:
- a container containing a lysis agent,
- an automated liquid handling system for autonomously adding lysis agent to at least two, preferably at least 8, most preferably at least 96 samples comprising bacterial cells,
wherein the lysis agent comprises a water-miscible ionic liquid containing choline.

12. Apparatus according to claim 10 or 11, wherein the ionic liquid further contains formate (Fmt), lactate (Lac), dibutylphosphate (DBP) or hexanoate (Hex), most preferably Hex.

13. A kit for NA amplification from a bacterial cell, the kit comprising:
- a lysis agent,
- a reagent for nucleic acid amplification, preferably selected from the group consisting of nucleoside triphosphates (NTPs), deoxynucleoside triphosphates (dNTPs), oligonucleotides, and NA amplification enzymes, preferably a DNA polymerase,
wherein the lysis agent comprises a water-miscible ionic liquid containing choline.

14. A kit for NA isolation from a bacterial cell, the kit comprising:
- a lysis agent,
- a solid support for the adsorption of a NA, the solid support preferably being a spin column, a bead, or a microchip or channel,
wherein the lysis agent comprises a water-miscible ionic liquid containing choline.

15. Kit according to claim 13 or 14, wherein the kit comprises an apparatus according to any one of claims 10 to 12.
